(19) Europäisches Patentamt
European Patent Office
Office européen des brevets

(11) Publication number: 0 023 761
B1

# (12) EUROPEAN PATENT SPECIFICATION

(45) Date of publication of patent specification: 07.03.84

(21) Application number: 80302140.1

(22) Date of filing: 26.06.80

(51) Int. Cl.[3]: C 07 D 513/04, C 07 D 498/04, A 61 K 31/47 //(C07D513/04 285/00, 221/00), (C07D498/04, 271/00, 221/00)

## (54) Tetrahydroisoquinoline derivatives, process for preparing them and compositions containing them.

(30) Priority: 02.07.79 US 54343

(43) Date of publication of application: 11.02.81 Bulletin 81/6

(45) Publication of the grant of the patent: 07.03.84 Bulletin 84/10

(84) Designated Contracting States: AT BE CH DE FR GB IT LI LU NL SE

(56) References cited:
US - A - 3 939 164
US - A - 3 988 339

(73) Proprietor: SMITHKLINE BECKMAN CORPORATION
1 Franklin Plaza
Philadelphia Pennsylvania 19101 (US)

(72) Inventor: Bondinell, William Edward
46 Curtis Avenue
Cherry Hill New Jersey 08002 (US)
Inventor: Girard, Gerald Robert
4741 LLanfair Place
Cornwells Heights Pennsylvania 19020 (US)

(74) Representative: Claisse, John Anthony, Dr. et al,
P.O. Box 39 Welwyn Garden City
Hertfordshire AL7 1EY (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 023 761 B1

Courier Press, Leamington Spa, England.

Tetrahydroisoquinoline derivatives, process for preparing them and compositions containing them

This invention relates to new thiadiazolo- and oxadiazolotetrahydroisoquinoline compounds. These compounds have pharmacological activity, in particular they inhibit the enzyme phenylethanolamine N-methyltransferase.

Epinephrine is a hormone, synthesized in the adrenal medulla, which is released into the blood stream in response to stress and produces profound physiological changes which serve to prepare the animal to cope with the stressor situation. For example, epinephrine produces anxiety, an increase in blood pressure, acceleration of heart rate and increase in cardiac output. These changes are detrimental in individuals with certain disease conditions such as angina pectoris, myocardial infarction and anxiety neuroses.

Phenylethanolamine N-methyltransferase catalyzes the final step in the biosynthesis of epinephrine, that is the transfer of a methyl group from S-adenosylmethionine to norepinephrine to produce epinephrine.

The compounds of this invention inhibit phenylethanolamine N-methyltransferase and thus reduce the formation of epinephrine. They are therefore useful in situations where there is overproduction of epinephrine or where epinephrine production is detrimental.

US—A—3939164 and US—A—3988339 disclose a variety of 7- and 8- substituted 1,2,3,4-tetrahydroisoquinolines which inhibit phenylethanolamine N-methyltransferase.

The compounds of this invention comprise the isomers having the following formulas:

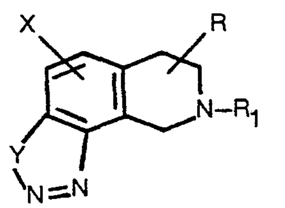

Formula I

;

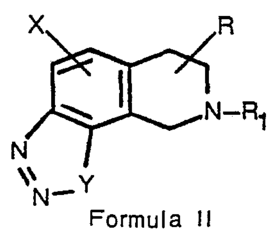

Formula II

;

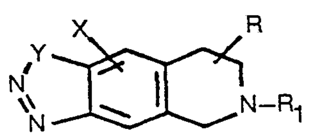

Formula III

;

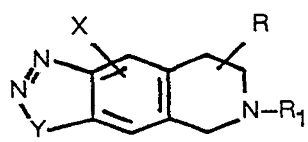

Formula IV

;

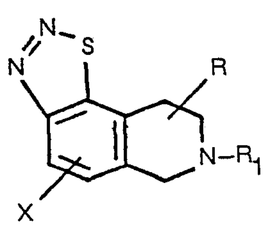

Formula V

; and

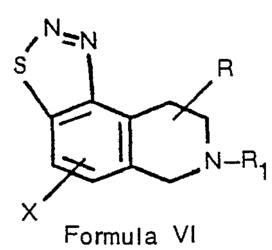

Formula VI

in which Y is sulfur or oxygen, X is hydrogen, halogen, or trifluoromethyl and R and $R_1$ which are the same or different are each hydrogen or $C_{1-3}$ alkyl of from one to three carbon atoms; provided that when Y is oxygen, X, R and $R_1$ are hydrogen; and pharmaceutically acceptable acid addition salts thereof.

Compounds of this invention represented by Formula I, when Y is sulfur R is $C_{1-3}$ alkyl and X and $R_1$ are both hydrogen, are prepared by the following procedure:

SCHEME I

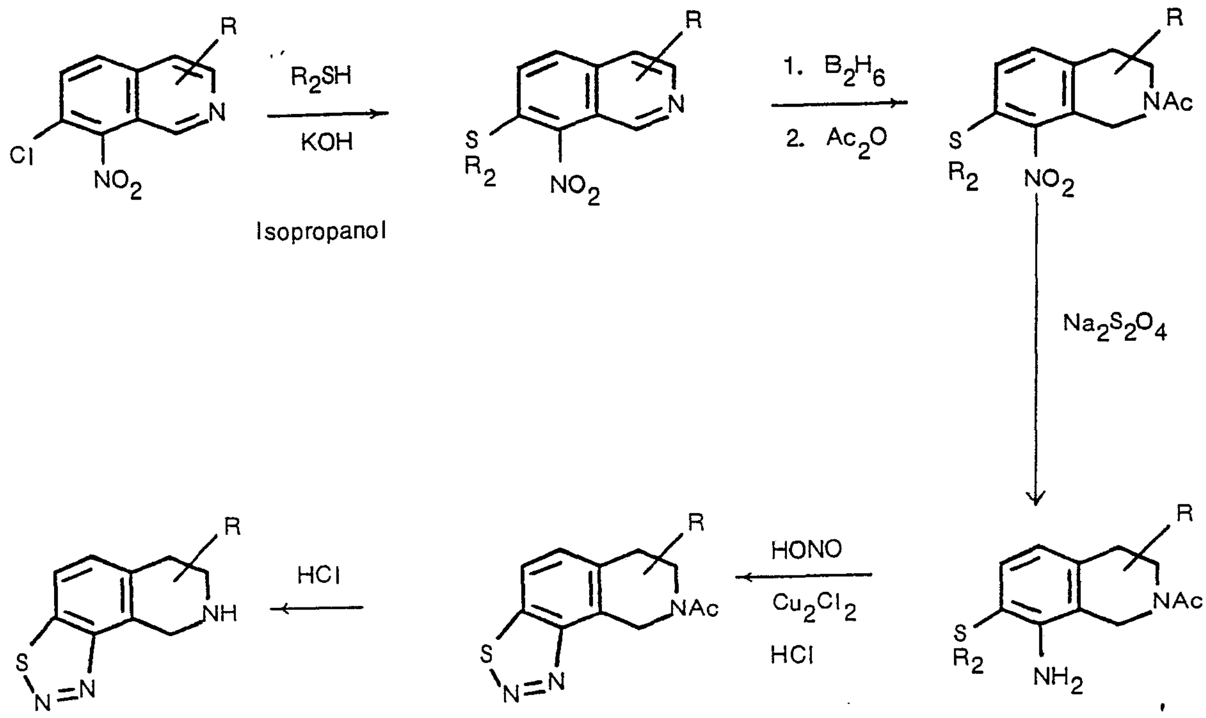


$R_2$ is any group capable of being displaced such as, for example, benzyl, tertiary butyl or isopropyl.

As shown above, the properly substituted 7-chloro-8-nitroisoquinoline is reacted with a mercaptan to yield the corresponding 7-thio derivative. The thio compound is then reduced with, for example, diborane and acylated to the corresponding N-acetyl tetrahydroisoquinoline. The nitro moiety is then reduced to the amine and cyclization is accomplished by diazotizing and treating the diazonium salt with cuprous chloride and hydrochloric acid. The acetyl group is then hydrolyzed.

A particular compound of this invention represented by Formula I when Y is sulfur and X, R, and $R_1$ are all hydrogen, being the compound [1,2,3]thiadiazolo[5,4-h]-6,7,8,9-tetrahydroisoquinoline, is also prepared by the above procedure.

When the isomeric form (Formula II) is desired, the properly substitued 8-chloro-7-nitroisoquinoline is employed as the starting material.

Compounds of Formulas III or IV where Y is sulfur are prepared from 2-acetyl-6-chloro or 2-acetyl-7-chloro-1,2,3,4-tetrahydroisoquinoline by nitration and treatment with a mercaptan to yield the respective thio compounds which are further converted by the methods shown in Scheme I, to give the desired thiadiazolo compounds.

Alternatively, compounds of Formulas I or II where Y is sulfur may be made from 8-amino- or 7-aminoisoquinoline, respectively, by treatment with sulfur monochloride to give a dithiazolo compound which is treated with nitrous acid to yield the desired thiadiazolo compound which is reduced, with, for example, sodium caynoborohydride as disclosed below.

SCHEME II

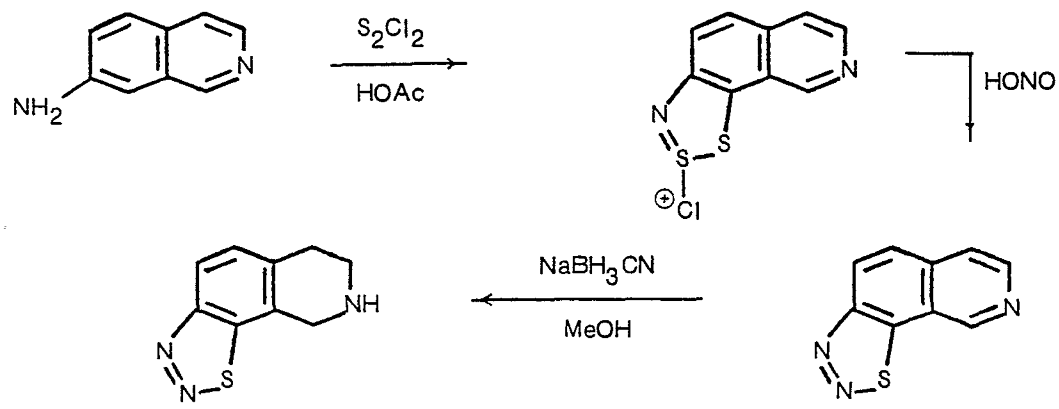


Formula II

Compounds of Formulas V and VI where Y is sulfur are prepared by treating 6-amino- or 5-aminoisoquinoline with sulfur monochloride followed by nitrous acid as shown in Scheme II.

The compounds where X is halogen are prepared by treating 5-amino or 8-aminoisoquinoline with sulfur monochloride. This results in the introduction of a chloro substituent into the vacant para-position to yield 5-chloro-[1,2,3]thiadiazolo[4,5-f]-6,7,8,9-tetrahydroisoquinoline and 5-chloro-[1,2,3]-thiadiazolo[5,4-h]-6,7,8,9-tetrahydroisoquinoline respectively.

Compounds of Formulas I—VI where $R_1$ is $C_{1-3}$ alkyl may be made by treatment of the corresponding tetrahydroisoquinoline where $R_1$ is hydrogen with the appropriate aldehyde or ketone followed by reaction with a reducing agent such as sodium cyanoborohydride.

The trifluoromethyl or halo derivatives may also be made by use of appropriately substituted starting materials and employing the procedures of Scheme II. For example, 7-amino-8-trifluoromethylisoquinoline and 7-amino-8-chloroisoquinoline are converted to 4-trifluoromethyl-[1,2,3]-thiadiazolo-[5,4-g]-5,6,7,8-tetrahydroisoquinoline and 4-chloro-[1,2,3]-thiadiazolo[5,4-g]-5,6,7,8-tetrahydroisoquinoline.

Oxadiazolotetrahydroisoquinolines of Formulas I and II, where Y is oxygen, are prepared from 2-acetyl-8(7)-amino-7(8)-chloro-1,2,3,4-tetrahydroisoquinoline and Formulas III and IV are prepared from 2-acetyl-7(6)-amino-6(7)-chloro-1,2,3,4-tetrahydroisoquinoline by diazotization and neutralization of the resulting solution to form the oxadiazolo moiety. The acetyl group is removed by hydrolysis.

The nontoxic pharmaceutically acceptable acid addition salts of the compounds of Formulas I—VI are similarly useful as the free bases. Such salts are easily prepared by methods known to the art. The base is reacted with an organic or inorganic acid in aqueous miscible solvent, such as acetone or ethanol, with isolation of the salt by concentration and cooling or in aqueous immiscible solvent, such as ethyl ether or chloroform, with the desired salt separating directly. Exemplary of the salts which are included in this invention are maleate, fumarate, benzoate, ascorbate, pamoate, succinate, bismethylenesalicylate, methanesulfonate, ethanedisulfonate, benzenesulfonate, acetate, propionate, tartrate, salicylate, citrate, gluconate, lactate, malate, mandelate, cinnamate, citraconate, aspartate, stearate, palmitate, itaconate, glycolate, p-aminobenzoate, glutamate, theophylline acetates, hydrochloride, hydrobromide, sulfate, cyclohexylsulfamate, phosphate and nitrate salts.

The activity of the compounds of Formulas I—VI is demonstrated by inhibition of phenylethanoline N-methyltransferase *in vitro* by the assay procedure described by Pendleton and Snow, *Molecular Pharmacology,* 9:718—725 (1973) at various compound concentrations. For example, at concentrations of $1.0 \times 10^{-4}$ and $1.0 \times 10^{-6}$ preferred compounds of this invention, [1,2,3]thiadiazolo[5,4-h]-6,7,8,9-tetrahydroisoquinoline hydrochloride and [1,2,3]thiadiazolo[4,5-h]-6,7,8,9-tetrahydroisoquinoline hydrochloride inhibit phenylethanolamine N-methyltransferase by 99% and 94% and by 99% and 91% respectively.

In addition, the activity of the compounds of this invention is demonstrated *in vivo* by administration to mice at 50 mg/kg per day for seven consecutive days. Male mice were dosed orally with either drug or vehicle control on a twice-a-day basis for seven consecutive days. On the morning of the next day they were again dosed and two hours later sacrificed by decapitation. The adrenal glands were then removed and analyzed fluorometrically for both epinephrine and norepinephrine content. A compound is considered active as a PNMT inhibitor if it significantly (at least $p < .05$) decreases the adrenal epinephrine/norepinephrine ratio (R. G. Pendleton et al., *J. Pharmacol. Exp. Ther.* 190:551—562, 1974 and R. G. Pendleton et al., *J. Pharmacol. Exp. Ther.* 197:623—632, 1976). An above preferred compound of this invention, [1,2,3]thiadiazolo[5,4-h]-6,7,8,9-tetrahydroisoquinoline hydrochloride, significantly lowered the adrenal epinephrine/norepinephrine ratio from $2.5 \pm 0.23$ to $1.3 \pm 0.08$ ($p < .01$).

The pharmaceutical compositions of this invention to inhibit phenylethanolamine N-methyl-transferase comprise a pharmaceutical carrier and, as the active ingredient, a tetrahydroisoquinoline compound of Formulas I—VI. The active ingredient will be present in the compositions of this invention in an effective amount to inhibit phenylethanolamine N-methyltransferase.

Preferably, the compositions of this invention contain the active ingredient of Formulas I—VI in an amount of from 50 mg to 1000 mg, advantageously from about 100 mg to about 500 mg, per dosage unit.

The pharmaceutical carrier can be a solid or liquid. Examples of solid carriers are lactose, magnesium stearate, terra alba, sucrose, talc, stearic acid, gelatin, agar, pectin and acacia. The amount of solid carrier will vary widely but preferably will be from 25 mg to 1 g. Examples of liquid carriers are syrup, peanut oil, olive oil, sesame oil, propylene glycol, polyethylene glycol (mol. wt. 200—400) and water. The carrier or diluent can include a time delay material well known to the art such as, for example, glyceryl monostearate or glyceryl distearate alone or with a wax.

A wide variety of pharmaceutical forms can be employed, for example, the compositions can take the form of: tablets, capsules, powders, troches, lozenges, syrups, emulsions, sterile injectable liquids or liquid suspensions or solutions.

The pharmaceutical compositions are prepared by conventional techniques involving procedures such as mixing, granulating and compressing or dissolving the ingredients as appropriate to the desired composition.

Phenylethanolamine N-methyltransferase can be inhibited by administering to an animal in an amount sufficient to inhibit phenylethanolamine N-methyl-transferase a tetrahydroisoquinoline compound of Formulas I—VI.

Preferably, the compounds of Formulas I—VI will be administered in conventional dosage unit forms prepared by combining an appropriate dose of the compound with standard pharmaceutical carriers.

Preferably, the active ingredient of Formulas 1—VI will be administered in a daily dosage regimen of from 100 mg to 2000 mg, most preferably from 200 mg to 1000 mg. Advantageously, equal doses will be administered preferably two to three times per day. When the administration is carried out as described above, inhibition of phenylethanolamine N-methyltransferase is produced.

The route of administration of the pharmaceutical compositions of this invention is internal, either parenteral or preferably oral, in an amount to produce the desired biological activity.

The following Examples are illustrative of the compounds of this invention, processes for their preparation, and composition containing them.

## Example 1

A mixture of 0.52 g (2.5 mmole) of 7-chloro-8-nitroisoquinoline and 0.317 g (2.56 mmole) of benzyl mercaptan in 5 ml of degassed isopropanol under argon at 0°C was treated with 0.16 g (2.5 mmole) of 86% KOH in 2 ml of ethanol dropwise over fifteen minutes. The mixture was stirred for one hour at 25°C and filterd. The collected product was washed with water and ethanol and then dried to yield 7-benzylthio-8-nitroisoquinoline having a melting point of 151—153°C.

A solution of 15 g (0.051 mole) of 7-benzylthio-8-nitroisoquinoline in 100 ml of tetrahydrofuran was added to 210 ml of 1M borane-tetrahydrofuran (0.21 mole). The mixture was stirred and refluxed for five hours. Methanol was added and the mixture evaporated in vacuo. The residue was treated with refluxing 12N HCl for 15 hours, then evaporated to dryness. The resulting solid was recrystallized from methanol-ether to give 7-benzylthio-8-nitro-1,2,3,4-tetrahydroisoquinoline hydrochloride having a melting point of 253°C with decomposition.

A mixture of 17.5 g (0.052 mole) of 7-benzylthio-8-nitro-1,2,3,4-tetrahydroisoquinoline hydrochloride, 15 ml of acetic anhydride and 4.5 g (0.055 mole) of sodium acetate in 150 ml of acetic acid was heated on a steam bath for one hour, then evaporated. Water and aqueous ammonia was added to the residue until the mixture was basic. The mixture was extracted with methylene chloride, the extracts combined and washed with water, 10% hydrochloric acid, 5% sodium bicarbonate, dried over sodium sulfate and concentrated. Chromatography of the residue on silica (ethyl acetate) gave 2-acetyl-7-benzylthio-8-nitro-1,2,3,4-tetrahydroisoquinoline, melting point 94—95°C.

A solution of 1.2 g (0.0035 mole) of the above 2-acetyl derivative in 15 ml of ethanol and 10 ml of water was treated with 4.2 g (0.23 mole) of sodium hydrosulfite. The mixture was refluxed for three hours, basified with aqueous ammonia and extracted with methylene chloride. The organic extracts were combined, washed with water, dried over sodium sulfate and concentrated to give 2-acetyl-8-amino-7-benzylthio-1,2,3,4-tetrahydroisoquinoline.

To a solution of 1.5 g (0.0048 mole) of the above 8-amino-tetrahydroisoquinoline derivative in 15 ml of 12N hydrochloric acid and 6 ml of acetic acid at −10°C under argon was added 0.52 g (0.0075 mole) of sodium nitrite in 3 ml of water. The mixture was stirred for five minutes then added rapidly to 2.3 g of cuprous chloride in 20 ml of 12N hydrochloric acid and heated to 60°C for three hours. The mixture was poured over ice, basified with aqueous ammonia and extracted with methylene chloride. The extracts were combined washed with water, dried and concentrated to give the corresponding diazonium salt. The salt was heated with 2.2 g of cuprous chloride and 40 ml of 12N hydrochloric acid for five hours at 60°C, followed by work-up as above to yield 8-acetyl[1,2,3]thiadiazolo[5,4-h]-6,7,8,9-tetrahydroisoquinoline having a melting point of 133—136°C.

A mixture of 0.15 g (0.0006 mole) of the above thiadiazolo derivative and 5 ml of 10% hydrochloric acid was refluxed for three hours and evaporated. The residue was dissolved in water, washed with methylene chloride, basified with aqueous ammonia and again extracted with methylene chloride. The methylene chloride extracts were combined, dried and evaporated. The residue was dissolved in methanol, acidified with hydrochloric acid-ether followed by further dilution with ether. The precipitate was recrystallized from methanol-ether to yield [1,2,3]thiadiazolo[5,4-h]-6,7,8,9-tetrahydroisoquinoline hydrochloride having a melting point of 285—286°C (dec.).

Example 2

A solution of 2.0 g (0.014 mole) of 7-aminoisoquinoline in 50 ml of acetic acid was added to 80 ml of cold sulfur monochloride and stirred for twenty-four hours. The mixture was filtered and the orange solid washed with ether and dried in vacuo to give [1,2,3]dithiazolo[4,5-h]isoquinolline-2-ium chloride hydrochloride, melting point 227—237°C.

A solution of 2.4 g (0.009 mole) of the above hydrochloride in 50 ml of 50% aqueous sulfuric acid was cooled to 0°C and treated with a solution of 0.97 g (0.014 mole) of sodium nitrite in 10 ml of water. The mixture was stirred at 0°C for two hours, poured into ice water, treated with charcoal, filtered, basified with ammonium hydroxide and extracted with ether. The combined ether extracts were dried, treated with charcoal, filtered and partly concentrated to yield [1,2,3]thiadiazolo[4,5-h]isoquinoline, melting point 150—153°C.

A solution of 0.5 g (0.003 mole) of the above isoquinoline in 50 ml of methanol was treated with 1 g (0.016 mole) of sodium cyanoborohydride and the resulting solution was stirred for twenty-four hours. The pH was maintained at pH 4 by the addition of methanolic hydrogen chloride. The mixture was then treated with excess methanolic hydrogen chloride and concentrated on a steam bath. The residue was dissolved in water, treated with charcoal, filtered and basified with ammonium hydroxide. The basic solution was extracted with ether and the combined extracts were dried, treated with charcoal and filtered. Treatment with ethereal hydrogen chloride gave [1,2,3]thiadiazolo[4,5-h]-6,7,8,9-tetrahydroisoquinoline hydrochloride, melting point 284—285°C.

Example 3

A solution of 20.9 g (0.1 mole) of 2-acetyl-7-chloro-1,2,3,4-tetrahydroisoquinoline in 100 ml of concentrated sulfuric acid is treated with 10 g (0.1 mole) of potassium nitrate dissolved in 150 ml of concentrated sulfuric acid. The mixture is stirred for one hour, quenched on ice, made alkaline with ammonium hydroxide and filtered to yield 2-acetyl-7-chloro-6-nitro-1,2,3,4-tetrahydroisoquinoline.

Treatment of the above tetrahydroisoquinoline with benzylmercaptan and following the procedure of Example 1 yields [1,2,3]thiadiazolo[4,5-g]-5,6,7,8-tetrahydroisoquinoline.

Example 4

Following the procedures of Example 3 and employing 2-acetyl-6-chloro-1,2,2,4-tetrahydroisoquinoline as a starting material yields 2-acetyl-6-chloro-7-nitro-1,2,3,4-tetrahydroisoquinoline which is converted to [1,2,3]thiadiazolo-[5,4-g]-5,6,7,8-tetrahydroisoquinoline.

Example 5

Following the procedure of Example 2, 8-aminoisoquinoline was converted to 5-chloro-[1,2,3]dithiazolo-[5,4-h]isoquinoline-2-ium chloride hydrochloride and then to 5-chloro-[1,2,3]thiadiazolo[5,4-h]isoquinoline, melting point, 225—227°C.

The above thiadiazoloisoquinoline is reduced with sodium cyanoborohydride also following the procedure of Example 2, to give 5-chloro-[1,2,3]thiadiazolo-[5,4-h]-6,7,8,9-tetrahydroisoquinoline.

Example 6

2-Acetyl-7-amino-8-chloro-1,2,3,4-tetrahydroisoquinoline (2.2 g, 0.01 mole) is dissolved in a mixture of 10 ml of concentrated hydrochloric acid and 20 ml of water and diazotized by the addition of sodium nitrite (0.7 g, 0.01 mole) at 3°C. The mixture is stirred for 30 minutes and then treated with sodium carbonate (7.0 g, 0.065 mole) to a pH of 7. The mixture is extracted with chloroform to give 8-acetyl-[1.2.3]oxadiazolo-[4,5-h]-6,7,8,9-tetrahydroisoquinoline on evaporation.

Refluxing with 10% hydrochloric acid, following the procedure of Example 1, gives [1,2,3]oxadiazolo-[4,5-h]-6,7,8,9-tetrahydroisoquinoline hydrochloride.

Example 7

Following the procedure of Example 6, 2-acetyl-8-amino-7-chloro-1,2,3,4-tetrahydroisoquinoline is converted to [1,2,3]-oxadiazolo[5,4-h]-6,7,8,9-tetrahydroisoquinoline hydrochloride.

Example 8

A mixture of 17.3 g (0.1 mole) of 7-methoxy-4-methylisoquinoline and 100 ml of 48% hydrobromic acid is refluxed for sixteen hours and evaporated in vacuo. The residue is dissolved in water, neutralized with ammonium hydroxide and filtered to yield 7-hydroxy-4-methylisoquinoline.

A mixture of 15.9 g (0.1 mole) of 7-hydroxy-4-methylisoquinoline and 41.7 g (0.125 mole) of triphenylphosphine dichloride is heated to 230°C. for four hours, cooled, and partitioned between chloroform and concentrated hydrochloric acid. The aqueous phase is neutralized with ammonium hydroxide and extracted with chloroform to yield 7-chloro-4-methylisoquinoline.

A mixture of 17.7 g (0.1 mole) of 7-chloro-4-methylisoquinoline and 100 ml of concentrated sulfuric acid is treated with 10 g (0.1 mole) of potassium nitrate dissolved in 150 ml of concentrated sulfuric acid. The mixture is stirred for one hour, quenched on ice, made alkaline with ammonium hydroxide and filtered to yield 7-chloro-4-methyl-8-nitroisoquinoline.

Following the procedure of Example 1, 7-chloro-4-methyl-8-nitroisoquinoline is converted to 6-methyl-[1,2,3]-thiadiazolo[5,4-h]-6,7,8,9-tetrahydroisoquinoline hydrochloride.

Example 9

A solution of 13.6 g (0.1 mole) of 3-methoxybenzaldehyde and 10.5 g (0.1 mole) of aminoacetaldehyde dimethyl acetal in 250 ml of toluene is refluxed for one hour and water produced in the reaction is collected in a Dean-Stark trap. The resulting solution of 3-methoxy-N-(2,2-dimethoxyethyl)benzylidenamine is added to a solution of (0.2 mole) methylmagnesium iodide in 250 ml of ether over 1.5 hours. The mixture is stirred for one hour, cooled and quenched by careful addition of 250 ml of water. The aqueous phase was filtered through Celite, extracted with 150 ml of ether and the combined organic phases washed, dried and evaporated to yield 3-methoxy-$\alpha$-methyl-N-(2,2-dimethoxyethyl)benzylamine.

A mixture of 23.9 g (0.1 mole) of 3-methoxy-$\alpha$-methyl-N-(2,2-dimethoxyethyl)benzylamine in 100 ml of dry pyridine is stirred, cooled and treated with a solution of 20.2 g (0.107 mole) of tosyl chloride in 100 ml of dry pyridine. The mixture is stirred at 25°C for three days, poured into 800 ml of water and extracted with ether. The combined ether extracts are washed with dilute hydrochloric acid and then with water, dried, filtered and evaporated to yield 3-methoxy-$\alpha$-methyl-N-(2,2-dimethoxyethyl)-N-tosyl-benzylamine.

A mixture of 39.3 g (0.1 mole) of 3-methoxy-$\alpha$-methyl-N-(2,2-dimethoxyethyl)-N-tosyl-benzylamine, one liter of dioxane and 80 ml of 6N hydrochloric acid is refluxed for six hours and allowed to stand for sixteen hours. The mixture is poured into two liters of water and extracted with ether. The aqueous phase is made alkaline with concentrated ammonium hydroxide and extracted with chloroform. The chloroform extract is washed, dried and evaporated to yield 7-methoxy-1-methylisoquinoline.

Following the procedures of Example 8, the above isoquinoline is converted to 9-methyl-[1,2,3]thiadiazolo-[5,4-h]-6,7,8,9-tetrahydroisoquinoline hydrochloride.

Example 10

A solution of 13.6 (0.1 mole) of 3-methoxybenzylamine and 11.8 g (0.1 mole) of pyruvaldehyde dimethyl acetal in 30 ml of toluene is refluxed for two hours and water formed in the reaction is collected in a Dean-Stark trap. The mixture is evaporated to yield the imine.

A mixture of 2.4 g (0.01 mole) of the imine and 0.08 g of platinum oxide in 200 ml of ethyl acetate is reduced in a hydrogen atmosphere to yield 3-methoxy-N-[2-(1,1-dimethoxypropyl)]benzylamine.

Following the procedures of Example 9, 3-methoxy-N-[2-(1,1-dimethoxypropyl)]benzylamine is converted to 7-methyl-[1,2,3]thiadiazolo[5,4-h]-6,7,8,9-tetrahydroisoquinoline hydrochloride.

Example 11

A solution of 1.9 g (0.01 mole) of [1,2,3]thiadiazolo[4,5-h]-6,7,8,9-tetrahydroisoquinoline and 4 ml of 37% aqueous formaldehyde in 15 ml of acetonitrile is treated with 1 g (0.016 mole) of sodium cyanoborohydride. The reaction mixture is stirred for fifteen minutes and then glacial acetic acid is added dropwise until the solution is neutral on wet pH paper. Stirring is continued for an additional forty-five minutes, acetic acid being added as needed to maintain the pH near neutrality. The solvent is evaporated, the residue is taken up in 2N aqueous potassium hydroxide and extracted with ether, which is washed, dried and evaporated to yield 8-methyl-[1,2,3]thiadiazolo[4,5-h]-6,7,8,9,-tetrahydroisoquinoline.

Example 12

Following the procedure of Example 2, 5-aminoisoquinoline and 6-aminoisoquinoline are converted, respectively, to 5-chloro-[1,2,3]thiadiazolo-[4,5-f]-6,7,8,9-tetrahydroisoquinoline and [1,2,3]thiadiazolo-[5,4-f]-6,7,8,9-tetrahydroisoquinoline.

Example 13

Following the procedure of Example 2, 7-amino-8-trifluoromethylisoquinoline and 7-amino-8-chloroisoquinoline are converted to 4-trifluoromethyl-[1,2,3]thiadiazolo-[5,4-g]-5,6,7,8-tetrahydroisoquinoline and 4-chloro-[1,2,3]-thiadiazolo[5,4-g]-5,6,7,8-tetrahydroisoquinoline, respectively.

Example 14

2-Acetyl-6-chloro-7-nitro-1,2,3,4-tetrahydroisoquinoline and 2-acetyl-7-chloro-6-nitro-1,2,3,4-tetrahydroisoquinoline are reduced with sodium hydrosulfite by the procedure of Example 1 to yield: 2-acetyl-7-amino-6-chloro-1,2,3,4-tetrahydroisoquinoline and 2-acetyl-6-amino-7-chloro-1,2,3,4-tetrachloroisoquinoline which are diazotized and neutralized following the procedure of Example 6 to yield: [1,2,3]-oxadiazolo[5,4-g]-5,6,7,8-tetrahydroisoquinoline and [1,2,3]-oxadiazolo-[4,5-g]-5,6,7,8-tetrahydroisoquinoline.

Example 15

| Ingredients | Amounts |
|---|---|
| [1,2,3]Thiadiazolo[5,4-h]-6,7,8,9-tetrahydroisoquinoline hydrochloride | 150 mg |
| Lactose | 350 mg |

The ingredients are mixed and filled into a hard gelatin capsule.

Example 16

| Ingredients | Amounts |
|---|---|
| [1,2,3]Oxadiaolo[5,4-h]-6,7,8,9-tetrahydroisoquinoline | 200 mg |
| Calcium sulfate dihydrate | 150 mg |
| Sucrose | 25 mg |
| Starch | 15 mg |
| Talc | 5 mg |
| Stearic Acid | 3 mg |

The calcium sulfate dihydrate, sucrose and the tetrahydroisoquinoline are thoroughly mixed and granulated with 10% gelatin solution. The wet granules are screened, dried and then mixed with the starch, talc and stearic acid, screened and compressed into a tablet.

One tablet is administered three times a day.

**Claims for the Contracting States: BE CH DE FR GB IT LI LU NL SE**

1. A compound of the formula:

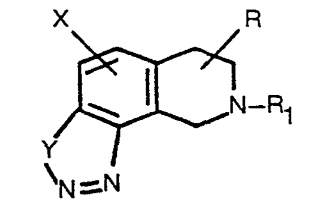 ; 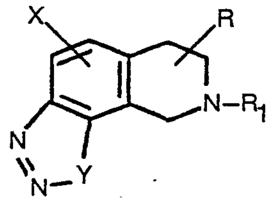 ;

Formula I

Formula II

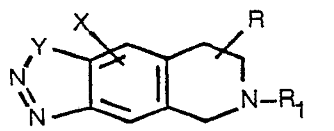 ; 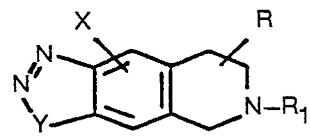 ;

Formula III

Formula IV

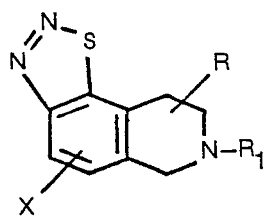 ; or 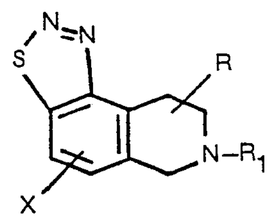

Formula V

Formula VI

in which Y is sulfur or oxygen, X is hydrogen, halogen, or trifluoromethyl, and R and $R_1$, which are the same or different, are hydrogen or $C_{1-3}$ alkyl provided that when Y is oxygen, X, R and $R_1$ are all hydrogen; or a pharmaceutically acceptable acid addition salt thereof.

2. A compound according to Claim 1 which is of the formula (I), (II), (III) or (IV).

3. A compound according to Claim 1, in which Y is sulfur.

4. A compound according to Claim 3, in which X, R and $R_1$ are all hydrogen and the compound is of Formula I or Formula II.

5. [1,2,3]-Thiadiazolo[5,4-h]-6,7,8,9-tetrahydroisoquinoline or a pharmaceutically acceptable acid addition salt thereof.

6. [1,2,3]-Thiadiazolo[4,5-h]-6,7,8,9-tetrahydroisoquinoline or a pharmaceutically acceptable addition salt thereof.

7. A compound according to Claim 3, in which X is halogen and the compound is of Formula I or Formula II.

8. 5-Chloro-[1,2,3]-thiadiazolo[5,4-h]-6,7,8,9-tetrahydroisoquinoline or a pharmaceutically acceptable addition salt thereof.

9. A compound according to Claim 3, in which X, R and $R_1$ are all hydrogen and the compound is of Formula III or Formula IV.

10. [1,2,3]-Thiadiazolo[5,4-g]-5,6,7,8-tetrahydroisoquinoline or a pharmaceutically acceptable addition salt thereof.

11. A compound according to Claim 1, in which X is trifluoromethyl, and R and $R_1$ are both hydrogen, and the compound is of Formula III or Formula IV.

12. A compound according to any of the preceding claims for use in inhibiting phenylethanolamine N-methyl-transferase.

13. A pharmaceutical composition comprising a pharmaceutical carrier and a compound according to any of the preceding claims.

14. A process for the preparation of a compound of the formula (I) to (VI) of claim 1 which process comprises:

i) for a compound of the formula (I), (II), (III), or (IV), the hydrolysis of a compound of the formula:

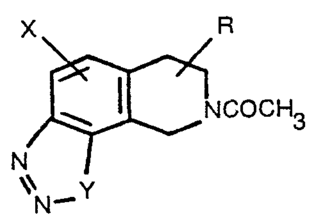

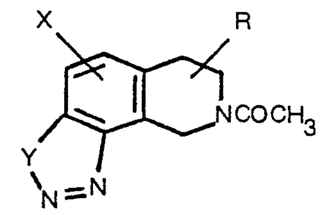

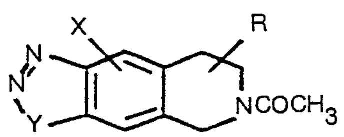

or

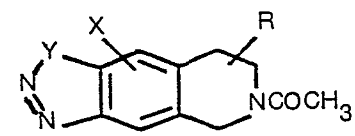

wherein R, X and Y are as defined in Claim 1; or

ii) for a compound of the formula (I), (II), (V) or (VI) wherein Y is sulphur, the reduction of a compound of the formula:

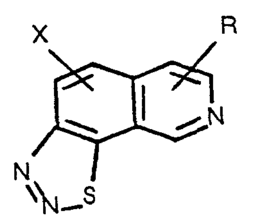

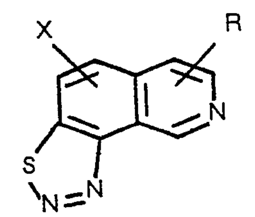

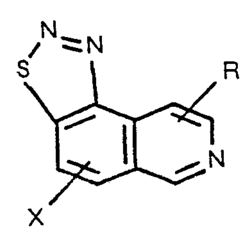

or

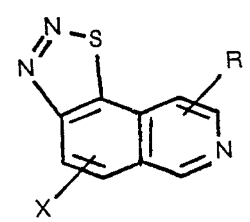

wherein R and X are as defined in Claim 1; and if necessary:

a) reaction with formaldehyde, acetaldehyde or propionaldehyde and subsequent reduction to form the N—$R_1$ moiety;

b) formation of a pharmaceutically acceptable acid addition salt.

15. A process for the preparation of a compound according to Claim 1 having the formula:

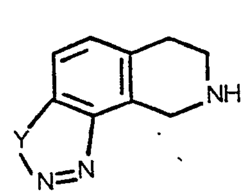

or

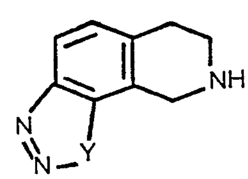

or a pharmaceutically acceptable acid addition salt thereof in which Y is sulfur or oxygen which comprises when Y is sulfur, cyclizing 2-acetyl-8(7)-amino-7(8)-$R_2$-thio-1,2,3,4-tetrahydroisoquinoline wherein $R_2$ is a displaceable group, by diazotization and hydrolyzing the acetyl group; and when Y is oxygen, diazotizing and neutralizing 2-actyl-8(7)-amino-7(8)-chloro-1,2,3,4-tetrahydroisoquinoline and hydrolyzing the acetyl group, and optionally reacting the product with an organic or inorganic acid to form the nontoxic pharmaceutically acceptable acid addition salts.

16. A process according to Claim 15 in which diazotization is accomplished with sodium nitrite in the presence of hydrochloric acid and $R_2$ is benzyl, tertiary butyl or isopropyl.

17. A process according to Claim 15 for the preparation of [1,2,3]thiazolo[5,4-h]-6,7,8,9-tetrahydroisoquinoline which comprises diazotizing 2-acetyl-8-amino-7-benzyl-thio-1,2,3,4-tetrahydroisoquinoline and hydrolyzing the acetyl group.

18. The process according to Claim 17 in which the product is reacted with hydrochloric acid to form the hydrochloride salt.

**Claims for the Contracting State: AT**

1. A process for the preparation of a compound of the formula:

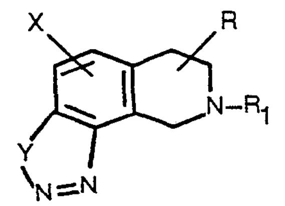

Formula I

;

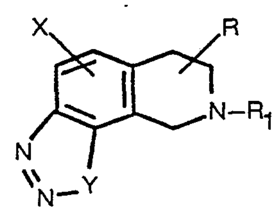

Formula II

;

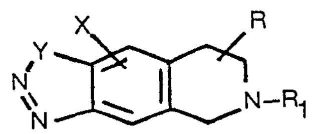

Formula III

;

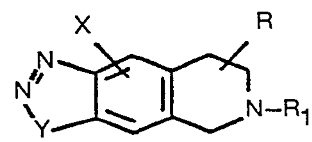

Formula IV

;

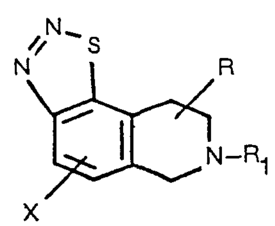

Formula V

; or

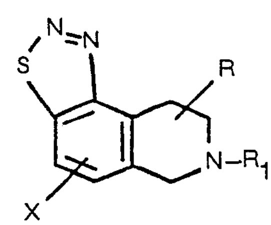

Formula VI

in which Y is sulfur or oxygen, X is hydrogen, halogen, or trifluoromethyl, and R and $R_1$, which are the same or different, are hydrogen or $C_{1-3}$ alkyl provided that when Y is oxygen, X, R and $R_1$ are all hydrogen; or a pharmaceutically acceptable acid addition salt thereof; which process comprises:

i) for a compound of the formula (I), (II), (III), or (IV), the hydrolysis of a compound of the formula:

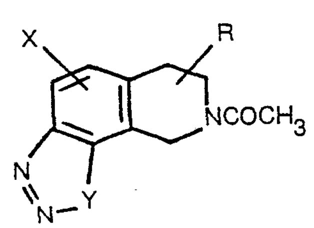

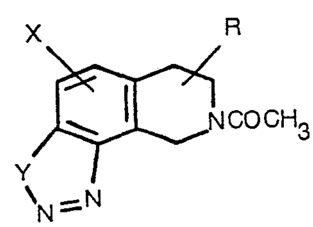

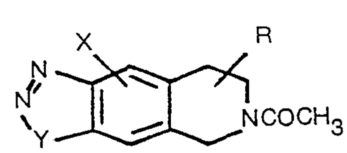

or

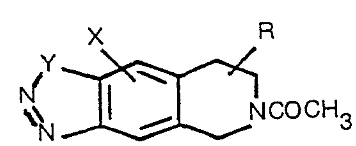

or

ii) for a compound of the formula (I), (II), (V) or (VI) wherein Y is sulphur, the reduction of a compound of the formula:

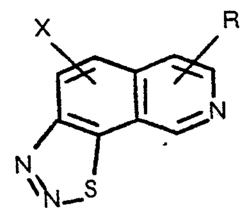 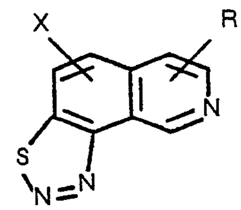

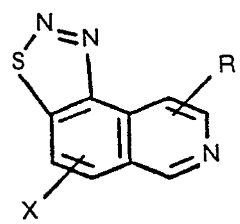 or 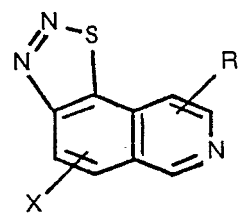

and if necessary:

a) reaction with formaldehyde, acetyaldehyde or propionaldehyde and subsequent reduction to form the N—$R_1$ moiety;

b) formation of a pharmaceutically acceptable acid addition salt.

2. A process for the preparation of a compound of the formula (I) or (II) as depicted in Claim 1, wherein X, R and $R_1$ are all hydrogen, which comprises when Y is sulphur, cyclizing 2-acetyl-8(7)-amino-7(8)-$R_2$thio-1,2,3,4-tetrahydroisoquinoline wherein $R_2$ is a displaceable group, by diazotising, and hydrolysing the acetyl group; and when Y is oxygen diazotising and neutralising 2-acetyl-8(7)-amino-7(8)chloro-1,2,3,4-tetrahydroisoquinoline and hydrolysing the acetyl group; and optionally reacting the product with an inorganic or organic acid to form a pharmaceutically acceptable acid addition salt.

3. A process according to Claim 2 wherein diazotisation is carried out with sodium nitrite in the presence of hydrochloric acid.

4. A process according to either Claim 2 or 3 wherein $R_2$ is benzyl, tertiary butyl or isopropyl.

5. A process according to Claim 2, 3 or 4 for the preparation of [1,2,3]-thiadiazolo[5,4-h]-6,7,8,9-tetrahydroisoquinoline which comprises diazotising 2-acetyl-8-amino-7-benzylthio-1,2,3,4-tetrahydroisoquinoline and hydrolysing the acetyl group.

6. A process according to any of claims 1, 2 or 5 wherein the product is reacted with hydrochloric acid to form the hydrochloride salt.

7. A process according to Claim 1 wherein the compound of formula (I), (II), (V) or (VI) is prepared by reduction of the isoquinoline ring with sodium cyano-borohydride.

**Revendications pour les Etats contractants: BE CH DE FR GB IT LI LU NL SE**

1. Composé de formule:

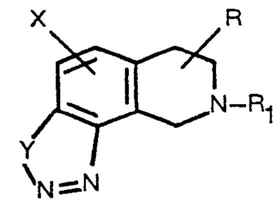

Formule I

;

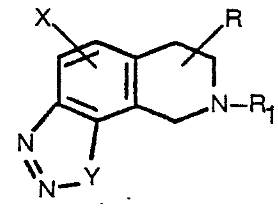

Formule II

;

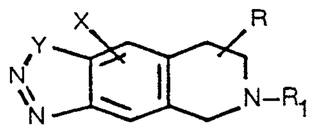

Formule III

;

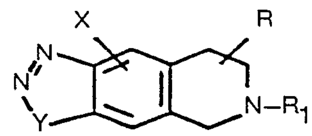

Formule IV

;

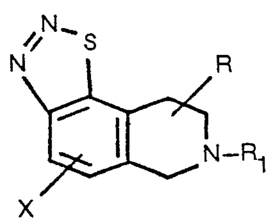

Formule V

; ou

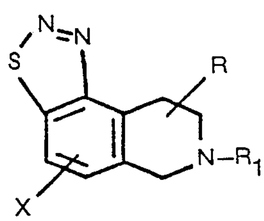

Formule VI

dans laquelle Y représente du soufre ou de l'oxygène, X représente de l'hydrogène, un halogène ou un groupe trifluorométhyle et R et $R_1$, qui sont identiques ou différents, représentent de l'hydrogène ou un groupe $C_{1-3}$ alcoyle à condition que lorsque Y représente de l'oxygène, X, R et $R_1$ représentent tous de l'hydrogène ou un sel d'addition d'acide pharmaceutiquement acceptable de ce composé.

2. Composé suivant la revendication 1, lequel est de la formule (I), (II), (III) our (IV).

3. Composé suivant la revendication 1, dans laquelle Y représente du soufre.

4. Composé suivant la revendication 3, dans laquelle X, R et $R_1$ représentent tous de l'hydrogène et le composé est de la formule I ou de la formule II.

5. La [1,2,3]-thiadiazolo[5,4-h]-6,7,8,9-tétrahydroisoquinoléine ou son sel d'addition avec les acides pharmaceutiquement acceptables.

6. La [1,2,3]-thiadiazolo[4,5-h]-6,7,8,9-tétrahydroisoquinoléine ou son sel d'addition avec les acides pharmaceutiquement acceptables.

7. Composé suivant la revendication 3, dans laquelle X représente un halogène et le composé est de la formule I ou de la formule II.

8. La 5-chloro-[1,2,3]thiadiazolo[4,5-h]-6,7,8,9-tétrahydroisoquinoléine ou son sel d'addition avec les acides pharmaceutiquement acceptables.

9. Composé suivant la revendication 3, dans laquelle X, R et $R_1$ représentent tous de l'hydrogène et le composé est de la formule III et de la formule IV.

10. La [1,2,3]-thiadiazolo[5,4-g]-5,6,7,8-tétrahydroisoquinoléine ou son sel d'addition avec les acides pharmaceutiquement acceptables.

11. Composé suivant la revendication 1, dans laquelle X représente un groupe trifluorométhyle et R et $R_1$ représentent tous deux de l'hydrogène et le composé est de la formule III ou de la formule IV.

12. Composé suivant l'une quelconque des revendications précédentes destiné à l'inhibition de la phényléthanolamine N-méthyltransférase.

13. Composition pharmaceutique comprenant un excipient pharmaceutique et un composé suivant l'une quelconque des revendications précédentes.

14. Procédé de préparation d'un composé de formule (I) à (VI) suivant la revendication 1, lequel procédé comprend:

i) pour un composé de formule (I), (II), (III) ou (IV), l'hydrolyse d'un composé de formule:

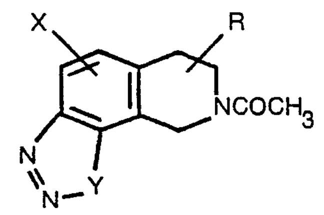

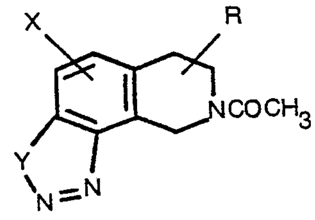

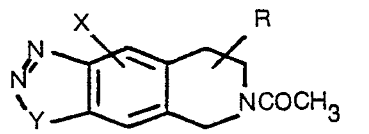

ou

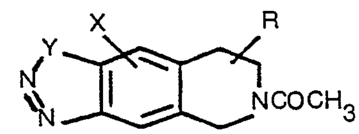

dans laquelle R, X et Y sont tels que définis dans la revendication 1 ou

ii) pour un composé de formule (I), (II), (V) ou (VI) dans laquelle Y représente du soufre, la réduction d'un composé de formule:

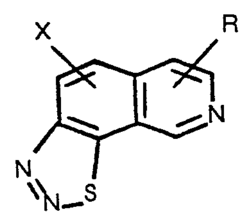

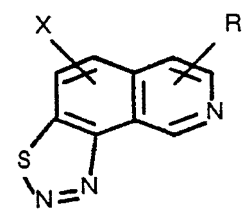

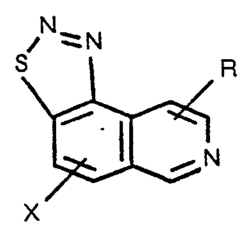

ou

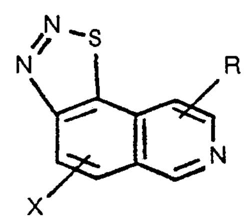

dans laquelle R et X sont tels que définis dans la revendication 1 et, le cas échéant:

a) la réaction avec le formaldéhyde, l'acétaldéhyde ou le propionaldéhyde et la réduction subséquente pour former le fragment N—$R_1$;

b) la formation d'un sel d'addition avec les acides pharmaceutiquement acceptables.

15. Procédé de préparation d'un composé suivant la revendication 1 et ayant la formula:

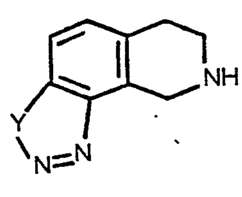

ou

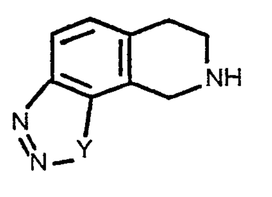

dans laquelle Y représente du soufre ou de l'oxygène, ou de son sel d'addition avec les acides pharmaceutiquement acceptables, qui comprend la cyclisation de la 2-acétyl-8(7)-amino-7(8)-$R_2$-thio-1,2,3,4-tétrahydroisoquinoléine, dans laquelle $R_2$ représente un groupe déplaçable, par diazotation et hydrolyse du groupe acétyle, lorsque Y représente du soufre et qui comprend la diazotation et la neutralisation de la 2-acétyl-8(7)-amino-7(8)-chloro-1,2,3,4-tétrahydroisoquinoléine et l'hydrolyse du group acétyle, lorsque Y représente de l'oxygène et la mise en réaction éventuelle du produit avec un acide organique ou minéral pour former des sels d'addition avec les acides pharmaceutiquement acceptables.

16. Procédé suivant la revendication 15, dans laquelle la diazotation est effectuée avec le nitrite de sodium en présence d'acide chlorhydrique et $R_2$ représente un groupe benzyle, butyle tertiaire ou isopropyle.

17. Procédé de préparation de la [1,2,3]thiadiazolo[5,4-h]-6,7,8,9-tétrahydroisoquinoléine suivant la revendication 15, qui comprend la diazotation de la 2-acétyl-8-amino-7-benzylthio-1,2,3,4-tétrahydroisoquinoléine et l'hydrolyse du groupe acétyle.

18. Procédé suivant la revendication 17, dans lequel on fait réagir le produit avec de l'acide chlorhydrique pour former le chlorhydrate.

**Revendications pour l'Etat contractant: AT**

1. Procédé de préparation d'un composé de formule:

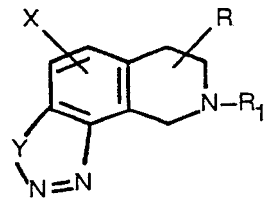

Formule I

;

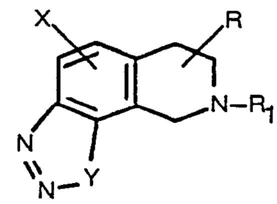

Formule II

;

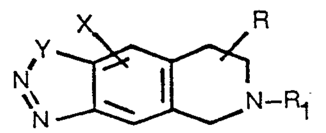

Formule III

;

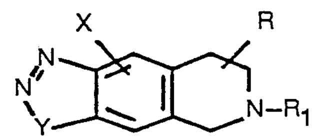

Formule IV

;

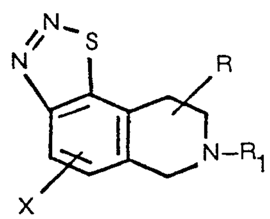

Formule V

; ou

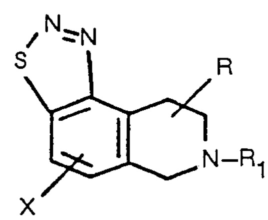

Formule VI

dans laquelle Y représente du soufre ou de l'oxygène, X représente de l'hydrogène, un halogène ou un groupe trifluorométhyle et R et $R_1$, qui sont identiques ou différents, représentent de l'hydrogène ou un groupe $C_{1-3}$ alcoyle à condition que lorsque Y représente de l'oxygène, X, R et $R_1$ représentent tous de l'hydrogène ou de son sel d'addition avec les acides pharmaceutiquement acceptables, lequel procédé comprend:

i) pour un composé de formule (I), (II), (III) ou (IV), l'hydrolyse d'un composé de formula:

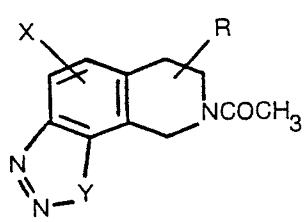

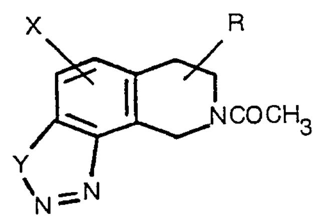

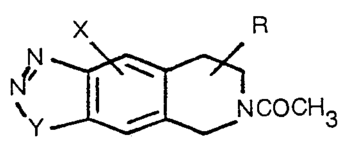

or

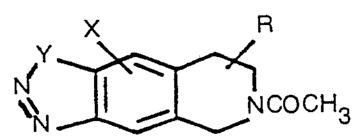

ou

ii) pour un composé de formule (I), (II), (V) ou (VI) dans laquelle Y représente du soufre, la réduction d'un composé de formule:

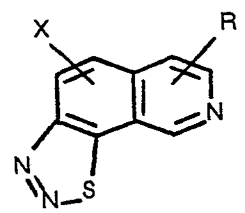

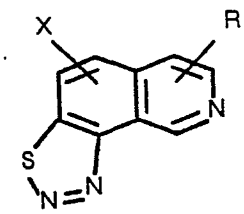

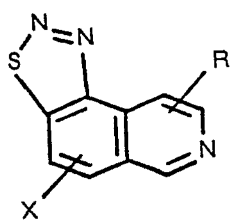

ou

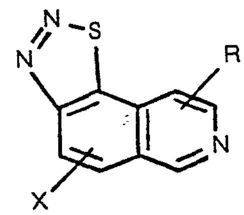

et, le cas échéant:

a) la réaction avec le formaldéhyde, l'acétaldéhyde ou le propionaldéhyde et la réduction subséquente pour former le fragment N—$R_1$;

b) la formation d'un sel d'addition avec les acides pharmaceutiquement acceptables.

2. Procédé de préparation d'un composé de formule (I) ou (II), telle que représentée dans la revendication 1, dans laquelle X, R et $R_1$ représentent tous de l'hydrogène, qui comprend la cyclisation de la 2-acétyl-8(7)-amino-7(8)-$R_2$-thio-1,2,3,4-tétrahydroisoquinoléine, dans laquelle $R_2$ représente un groupe deplaçable, par diazotation et hydrolyse du groupe acétyle, lorsque Y représente du soufre et qui comprend la diazotation et la neutralisation de la 2-acétyl-8(7)-amino-7(8)-chloro-1,2,3,4-tétrahydroisoquinoléine et l'hydrolyse du groupe acétyle, lorsque Y représente de l'oxygène et la mise en réaction éventuelle du produit avec un acide organique ou minéral pour former un sel d'addition avec les acides pharmaceutiquement acceptable.

3. Procédé suivant la revendication 2, dans laquelle la diazotation est effectuée avec le nitrite de sodium en présence d'acide chlorhydrique.

4. Procédé suivant l'une ou l'autre revendications 2 ou 3 dans laquelle $R_2$ représente un groupe benzyle, butyle tertiaire ou isopropyle.

5. Procédé de préparation de la [1,2,3]thiadiazolo[5,4-h]-6,7,8,9-tétrahydroisoquinoléine suivant la revendication 2, 3 ou 4, qui comprend la diazotation de la 2-acétyl-8-amino-7-benzylthio-1,2,3,4-tétrahydroisoquinoléine et l'hydrolyse du groupe acétyle.

6. Procédé suivant l'une quelconque des revendications 1, 2 ou 5, dans lequel on fait réagir le produit avec de l'acide chlorhydrique pour former le chlorhydrate.

7. Procédé suivant la revendication 1, dans lequel on prépare un composé de formule (I), (II), (V) ou (VI) par réduction du noyau isoquinoléinique avec le cyanoborohydrure de sodium.

**Patentansprüche für die Vertragsstaaten: BE CH DE FR GB IT LI LU NL SE**

1. Eine Verbindung der Formel:

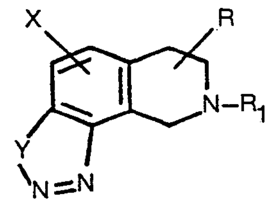

Formel I

;

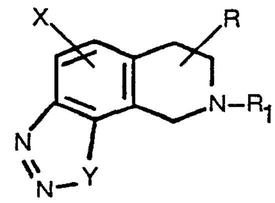

Formel II

;

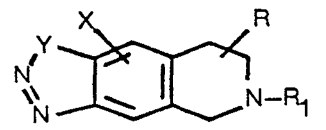

Formel III

;

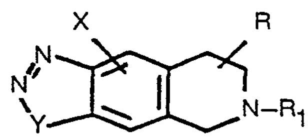

Formel IV

;

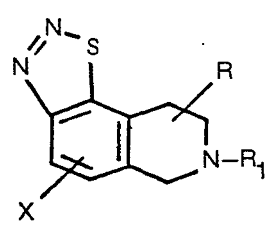

Formel V

; oder

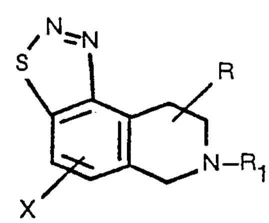

Formel VI

in der Y Schwefel oder Sauerstoff ist, X Wasserstoff, Halogen oder Trifluormethyl ist und R und $R_1$ gleich oder verschieden sind und Wasserstoff oder $C_{1-3}$-Alkyl bedeuten mit der Maßgabe, daß X, R und $R_1$ jeweils ein Wasserstoffatom bedeuten, falls Y Sauerstoff ist; oder ihr pharmazeutisch verträgliches Säureadditionssalz.

2. Eine Verbindung nach Anspruch 1 der Formel (I), (II), (III) oder (IV).

3. Eine Verbindung nach Anspruch 1, wobei Y Schwefel ist.

4. Eine Verbindung nach Anspruch 3, wobei X, R und $R_1$ jeweils Wasserstoffatome bedeuten und die Verbindung der Formel I oder der Formel II entspricht.

5. [1,2,3]-Thiadiazolo[5,4-h]-6,7,8,9-tetrahydroisochinolin oder sein pharmazeutisch verträgliches Säureadditionssalz.

6. [1,2,3]-Thiadiazolo[4,5-h]-6,7,8,9-tetrahydroisochinolin oder sein pharmazeutisch verträgliches Säureadditionssalz.

7. Eine Verbindung nach Anspruch 3, wobei X Halogen ist und die Verbindung der Formel I oder der Formel II entspricht.

8. 5-Chlor-[1,2,3]-thiadiazolo[4,5-h]-6,7,8,9-tetrahydroisochinolin oder sein pharmazeutisch verträgliches Säureadditionssalz.

9. Eine Verbindung nach Anspruch 3, wobei X, R und $R_1$ jeweils Wasserstoffatome bedeuten und die Verbindung der Formel III oder der Formel IV entspricht.

10. [1,2,3]-Thiadiazolo[5,4-g]-5,6,7,8-tetrahydroisochinolin oder sein pharmazeutisch verträgliches Säureadditionssalz.

11. Eine Verbindung nach Anspruch 1, wobei X Trifluormethyl ist und R und $R_1$ jeweils Wasserstoffatome bedeuten, und die Verbindung der Formel III oder der Formel IV entspricht.

12. Eine Verbindung nach einem der vorstehenden Ansprüche zur Verwendung bei der Inhibierung von Phenyläthanolamin-N-Methyltransferase.

13. Arzneimittel, enthaltend einen pharmazeutischen Trägerstoff und eine Verbindung nach einem der vorstehenden Ansprüche.

14. Ein Verfahren zur Herstellung einer Verbindung der Formeln (I) bis (VI) nach Anspruch 1, dadurch gekennzeichnet, daß man

i) zur Herstellung einer Verbindung der Formel (I), (II), (III) oder (IV) einer Verbindung der Formel:

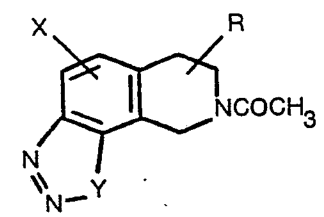

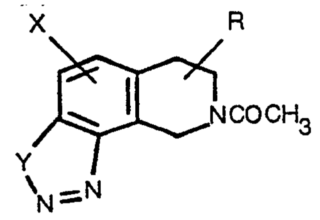

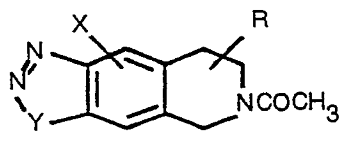

oder

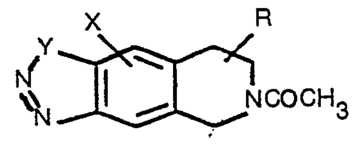

in der R, X und Y die in Anspruch 1 angegebene Bedeutung haben, hydrolysiert, oder

ii) zur Herstellung einer Verbindung der Formel (I), (II), (V) oder (VI), in der Y Schwefel ist, eine Verbindung der Formel:

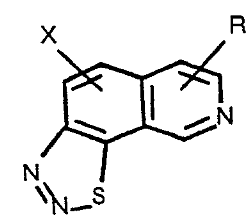

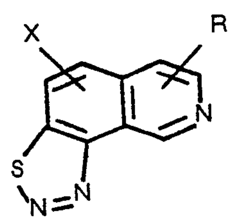

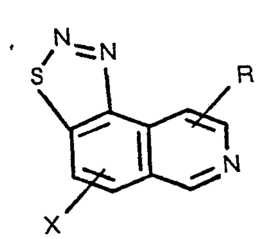

oder

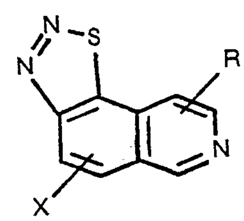

in der R und X in Anspruch 1 angegebene Bedeutung haben, reduziert, und, falls erforderlich,

a) die erhaltene Verbindung mit Formaldehyd, Acetaldehyd oder Propionaldehyd umsetzt und anschließend unter Bildung des N—$R_1$ Restes reduziert;

b) die erhaltene Verbindung in ihr pharmazeutisch verträgliches Säureadditionssalz überführt.

15. Ein Verfahren zur Herstellung einer Verbindung nach Anspruch 1 der Formel:

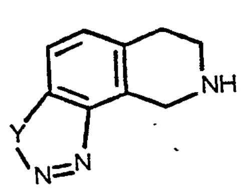

oder

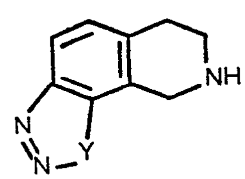

oder ihr pharmazeutisch verträgliches Säureadditionssalz, wobei Y Schwefel oder Sauerstoff ist, dadurch gekennzeichnet, daß man, falls Y eine Schwefelatom bedeutet, 2-Acetyl-8(7)-amino-7(8)-$R_2$-thio-1,2,3,4-tetrahydroisochinolin, in dem $R_2$ einen verdrängbaren Rest darstellt, diazotiert und die Acetylgruppe hydrolytisch spaltet; und, falls Y ein Sauerstoffatom bedeutet, 2-Acetyl-8(7)-amino-7(8)-chlor-1,2,3,4-tetrahydroisochinolin diazotiert und neutralisiert und die Acetylgruppe hydrolytisch abspaltet, und gegebenenfalls die erhaltene Verbindung mit einer organischen oder anorganischen Säure in das nicht-toxische pharmazeutisch verträgliche Säureadditionssalz überführt.

16. Ein Verfahren nach Anspruch 15, bei dem die Diazotierung mit Natriumnitrit in Gegenwart von Salzsäure durchgeführt wird, wobei $R_2$ Benzyl, tert.-Butyl oder Isopropyl bedeutet.

17. Ein Verfahren nach Anspruch 15 zur Herstellung von [1,2,3]-Thiadiazolo[5,4-h]-6,7,8,9-tetrahydroisochinolin, dadurch gekennzeichnet, daß man 2-Acetyl-8-amino-7-benzylthio-1,2,3,4-tetrahydroisochinolin diazotiert und die Acetylgruppe hydrolytisch abspaltet.

18. Das Verfahren nach Anspruch 17, bei dem die erhaltene Verbindung mit Salzsäure in das Hydrochlorid umgewandelt wird.

**Patentansprüche für den Vertragsstaat: AT**

1. Ein Verfahren zur Herstellung einer Verbindung der Formel:

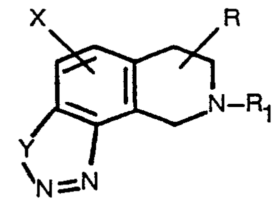

Formel I

;

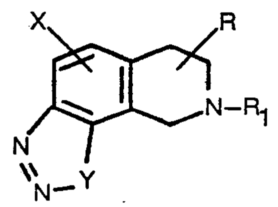

Formel II

;

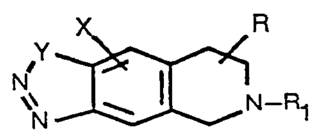

Formel III

;

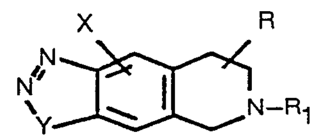

Formel IV

;

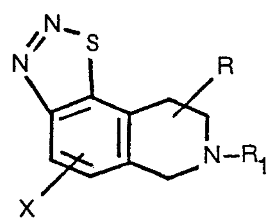

Formel V

; oder

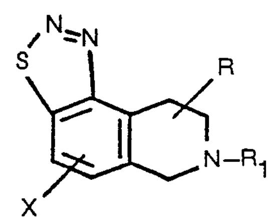

Formel VI

in der Y Schwefel oder Sauerstoff ist, X Wasserstoff, Halogen oder Trifluormethyl ist und R und $R_1$ gleich oder verschieden sind und Wasserstoff oder $C_{1-3}$-Alkyl bedeuten, mit der Maßgabe, daß, falls Y ein Sauerstoffatom bedeutet, X, R und $R_1$ jeweils Wasserstoffatome sind, oder ihr pharmazeutisch verträgliches Säureadditionssalz; dadurch gekennzeichnet, daß man

i) zur Herstellung einer Verbindung der Formel (I), (II), (III) oder (IV) eine Verbindung der Formel:

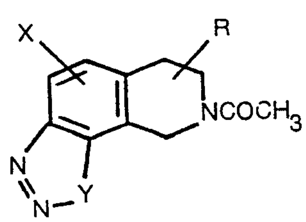

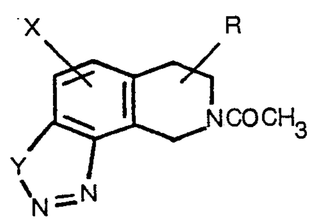

,

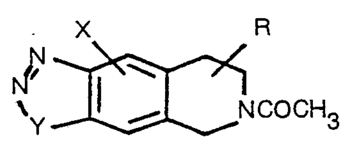

oder

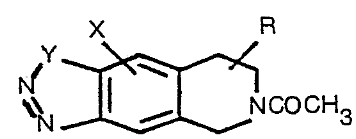

hydrolysiert, oder

ii) zur Herstellung einer Verbindung der Formel (I), (II), (V) oder (VI), in der Y Schwefel ist, eine Verbindung der Formel:

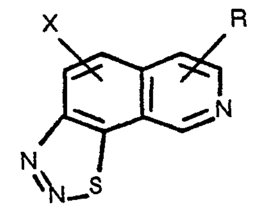

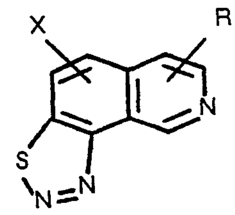

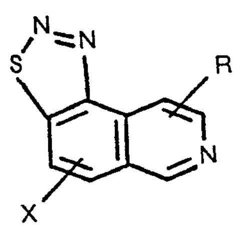

oder

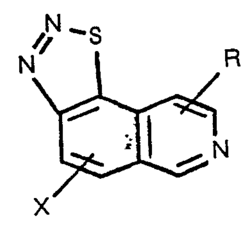

reduziert,

und, falls erforderlich,

a) die erhaltene Verbindung mit Formaldehyd, Acetaldhyd oder Propionaldehyd umsetzt und anschließend unter bildung des N—$R_1$ Restes reduziert;

b) die erhaltene Verbindung in ihr pharmazeutisch verträgliches Säureadditionssalz überführt.

2. Ein Verfahren zur Herstellung einer Verbindung der Formel (I) oder (II) nach Anspruch 1, wobei X, R und $R_1$ jeweils Wasserstoffatome bedeuten, dadurch gekennzeichnet, daß man, falls Y ein Schwefelatom bedeutet, 2-Acetyl-8(7)-amino-7(8)-$R_2$-thio-1,2,3,4-tetrahydroisochinolin, in dem $R_2$ einen verdrängbaren Rest darstellt, durch Diazotieren cyclisiert und die Acetylgruppe hydrolytisch spaltet; und, falls Y eine Sauerstoffatom bedeutet, 2-Acetyl-8(7)-amino-7(8)-chlor-1,2,3,4-tetrahydroisochinolin diazotiert und neutralisiert und die Acetylgruppe hydrolytisch abspaltet, und gegebenenfalls die erhaltene Verbindung mit einer organischen oder anorganischen Säure in ein pharmazeutisch verträgliches Säureadditionssalz überführt.

3. Ein Verfahren nach Anspruch 2, bei dem die Diazotierung mit Natriumnitrit in Gegenwart von Salzsäure durchgeführt wird, wobei $R_2$ Benzyl, tert.-Butyl oder Isopropyl bedeutet.

4. Ein Verfahren nach einem der Ansprüche 2 oder 3, wobei $R_2$ Benzyl, tert.-Butyl oder Isopropyl ist.

5. Ein Verfahren nach Anspruch 2, 3 oder 4 zur Herstellung von [1,2,3]-Thiadiazolo[5,4-h]-6,7,8,9-tetrahydroisochinolin, dadurch gekennzeichnet, daß man 2-Acetyl-8-amino-7-benzyl-thio-1,2,3,4-tetrahydroisochinolin diazotiert und die Acetylgruppe hydrolytisch abspaltet.

6. Ein Verfahren nach einem der Ansprüche 1, 2 oder 5, bei dem die erhaltene Verbindung mit Salzsäure in das Hydrochlorid umgewandelt wird.

7. Ein Verfahren nach Anspruch 1, wobei die Verbindung der Formel (I), (II), (V) oder (VI) durch Reduktion des Isochinolinringes mit Natriumcyanoborhydrid hergestellt wird.